# EUROPEAN PATENT SPECIFICATION

(11) **EP 3 762 078 B1**
(45) Date of publication and mention of the grant of the patent: **30.08.2023**
(21) Application number: 19764074.1
(22) Date of filing: 18.02.2019
(51) Int. Cl.: A61M 16/06, A61M 16/08, A61M 16/10, A61B 5/08, A61B 5/097, A61M 16/00, A61M 16/04, A61B 5/083

(54) **CAPNOGRAPHY FITTING**
CAPNOGRAPHIE-ARMATUR
RACCORD DE CAPNOGRAPHIE

(30) Priority: 09.03.2018 US 201862641011 P
(43) Date of publication of application: 13.01.2021
(73) Proprietor: Hu-Friedy Mfg. Co., LLC, Chicago, IL 60618-5935 (US)
(72) Inventor: VANPELT, Benjamin F., Peoria, Arizona 85383 (US)
(74) Representative: FRKelly
(86) International application number: PCT/US2019/018415
(87) International publication number: WO 2019/173038

(56) References cited:
- EP-B1- 1 684 720
- CA-A1- 2 274 398
- DE-U1-202012 007 387
- GB-A- 2 479 823
- US-A- 3 490 496
- US-A- 4 508 533
- US-A- 5 501 669
- US-A- 6 135 109
- US-A- 6 135 109
- US-A1- 2004 080 155
- US-A1- 2007 240 709
- US-A1- 2010 071 688
- US-B2- 6 929 008

## Description

### BACKGROUND

Capnography is a non-invasive monitoring method used to measure carbon dioxide concentration in the exhaled breath of a patient. Measurements of the carbon dioxide concentration in a patient's breath are taken by a capnography device and the results are displayed in a graphical format in the shape of a waveform called a capnogram. Typically, capnograms depict carbon dioxide concentration over time.

Capnography devices have been used in various healthcare fields for some time. The amount of carbon dioxide exhaled from a patient's breath is a useful measurement to determine how well a patient is breathing especially when an anesthetic, analgesic or other medication is used to sedate the patient. For example, if the patient has reduced breathing during sedation, carbon dioxide will not be able to be exhaled by the patient. This lack of exhalation of carbon dioxide will make the capnogram show a low carbon dioxide reading, which will trigger an alarm that alerts medical staff to the problem. In this way, a compromised respiratory system can be detected early in the medical or dental procedure.

Typically, capnography is performed in conjunction with an inhalation system for administering inhalation gas to a patient such inhalation gas includes oxygen and/or an anesthetic agent. Often, inhalation systems include a source of the inhalation gas and an apparatus to deliver the gas to the respiratory system of the patient. A breathing device, such as a disposable inhalation mask, is fitted on the face of the patient to cover the nose and/or the mouth. The inhalation gas source, which may be either portable or fixed, usually includes a flow regulator. A delivery conduit, generally in the form of a flexible hose, communicates between the inhalation gas source and the breathing device.

The inhalation system may include a capnography scavenging apparatus having a sampling tube and a return conduit extending from the breathing device to a vacuum source.

When the inhalation system is used and the patient is breathing, the sampling tube contains a sample of the air from the patient's breathing during the medical or dental procedure. The capnography scavenging apparatus is coupled to the capnography device, which produces a graphical read out of the carbon dioxide contained in the patient's exhaled gas.

Generally, the capnography scavenging apparatus is fixed to the inhalation mask and changing the location of the sampling tube cannot be done simply. By fixing the capnography scavenging apparatus including the sampling tube to one location, it can prevent the apparatus from obtaining an optimal reading from the patient. Further, the fixed apparatus cannot be adapted from a right-handed user to a left-handed user or from a left-handed user to a right-handed user easily. This can make using the capnography scavenging apparatus limited and not versatile for multiple users.

Currently, in order to change the location of the sampling tube of the capnography scavenging apparatus, a user, such as a clinician, has to manually punch hole(s) into the wall(s) of the mask in order to make changes to the mask's dynamics. This can be a crude task and difficult to perform either before or during the medical or dental procedure. Additionally, movement of the sampling tube becomes cumbersome. Further, changing the location of the sampling tube in this manner is not time efficient and the hole created by the user may cause the mask to no longer have an air tight seal, which may further disrupt gas flow and may lead to less accurate capnography readings as gas escapes from the seal.

Therefore, there is a need for a capnography fitting that is suitable for use with both right handed and left-handed users. There is also a need for an improved capnography fitting that allows the sampling tube to be easily inserted into and removed from the desired location in the inhalation mask. A capnography fitting that fits inhalation masks of various sizes and shapes would be beneficial.

Document US 2007/240709 discloses an entrainment device comprising a coupler with an angled port at an acute angle relative to a surface of the proximal end inlet of the tube.

### SUMMARY

The invention is defined in independent claim 1. Preferred embodiments are disclosed in the dependent claims.

A capnography fitting is provided that is compatible with an inhalation mask and can easily be inserted by both right handed and left-handed users. The capnography fitting provided fits inhalation masks of various sizes and shapes. In some embodiments, the capnography fitting provided allows the sampling tube to be easily inserted into and removed from the desired location in the inhalation mask.

In some embodiments, there is a capnography fitting comprising a first tube having a proximal end inlet and a distal end outlet and a second tube adjacent to and extending substantially parallel to the first tube. The first tube having an angled port in fluid communication with and disposed adjacent to the distal end outlet of the first tube. The second tube having a diameter smaller than a diameter of the first tube.

In some embodiments, there is a capnography fitting comprising a first tube having a proximal end inlet and a distal end outlet. The first tube has an angled port in fluid communication with and disposed adjacent to the proximal end inlet and the distal end outlet. The distal end outlet is configured to engage an inlet of a nasal mask. A second tube is provided having a proximal end outlet and a distal end inlet, the second tube adjacent to and extending substantially parallel to the first tube and having a diameter smaller than a diameter of the first tube. The distal end inlet is configured to engage an outlet of a nasal mask. The capnography fitting comprises a support attached to the first tube and the second tube.

In some embodiments, a kit for capnography monitoring is provided. The kit comprises a capnography fitting comprising a first tube having a proximal end inlet and a distal end outlet, the first tube having an angled port in fluid communication with and disposed adjacent to the proximal end inlet and the distal end outlet, a second tube having a proximal end outlet and a distal end inlet, the second tube adjacent to and extending substantially parallel to the first tube and having a diameter smaller than a diameter of the first tube, and a support attached to the first tube and the second tube; and instructions for assembling the kit for capnography monitoring.

In some embodiments, a method of assembling a capnography system is provided. The method comprises: providing a capnography fitting, the capnography fitting comprising a first tube having a proximal end inlet and a distal end outlet, the first tube having an angled port in fluid communication with and disposed adjacent to the proximal end inlet and the distal end outlet, a second tube having a proximal end outlet and a distal end inlet, the second tube adjacent to and extending substantially parallel to the first tube and having a diameter smaller than a diameter of the first tube, and a support attached to the first tube and the second tube; inserting the proximal end inlet of the first tube and the proximal end outlet of the second tube into a sleeve; and coupling the distal end outlet of the first tube to an inlet of a nasal mask; and coupling the distal end inlet of the second tube to an outlet of the nasal mask to assemble the capnography system.

In some embodiments, a method of monitoring a patient's respiratory functions is provided. The method comprising: attaching a nasal mask to a nose of the patient, the nasal mask having an inlet to receive inhalation gas and an outlet to discharge exhalation gas, the nasal mask comprising a capnography fitting comprising a first tube having a proximal end inlet and a distal end outlet, the first tube having an angled port in fluid communication with and disposed adjacent to the proximal end inlet and the distal end outlet, a second tube having a proximal end outlet and a distal end inlet, the second tube adjacent to and extending substantially parallel to the first tube and having a diameter smaller than a diameter of the first tube, and a support attached to the first tube and the second tube, the distal end outlet of the first tube slidably engaging the inlet of the nasal mask and the distal end inlet of the second tube slidably engaging the outlet of the nasal mask; providing inhalation gas to the patient; and obtaining a sample of the exhalation gas from the angled port to monitor the patient's respiratory functions.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### BRIEF DESCRIPTION OF THE FIGURES

In part, other aspects, features, benefits and advantages of the embodiments will be apparent with regard to the following description, appended claims and accompanying drawings where:
FIG. 1 illustrates a perspective view of an embodiment of a capnography fitting having an angled port for use with an inhalation mask, such as for example, a nasal mask;
FIG. 2 illustrates a perspective view of the capnography fitting of FIG. 1;
FIG. 3 illustrates a side view of the capnography fitting of FIG. 1;
FIG. 4 illustrates a cross sectional view of the capnography fitting of FIG. 1;
FIG. 5 illustrates an exploded view of components for use with the capnography fitting of FIG. 1;
FIG. 6 illustrates a perspective view of the components of FIG. 5;
FIG. 7 illustrates a perspective view of the components of FIG. 5 connected to the capnography fitting of FIG. 1;
FIG. 7A illustrates a perspective view of the components of FIG. 5 connected to the capnography fitting of FIG. 1, without the sampling tube;
FIG. 8 illustrates a side view of a component for use in a capnography system, as set forth and described herein;
FIG. 9 illustrates a side view of components for use in a capnography system, as set forth and described herein;
FIG. 10 illustrates a perspective view of components of a capnography system including the capnography fitting of FIG. 1;
FIG. 11 illustrates a perspective view of components of a capnography system including the capnography fitting of FIG. 1;
FIG. 11A illustrates a perspective view of the components of a capnography system including the capnography fitting of FIG. 1, without the luer fitting/internally threaded cap and showing an interference fitting, press fitting or friction fitting between the sampling tube and the angled port;
FIG 12 illustrates a perspective view of components of a capnography system including the capnography fitting of FIG. 1;
FIG. 13 illustrates a perspective view of components of a capnography system including the capnography fitting of FIG. 1; and
FIG. 14 illustrates a perspective view of a capnography system including the capnography fitting of FIG. 1 attached to a capnography monitor.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding the numerical ranges and parameters set forth herein, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, *e.g.*, 5.5 to 10.

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents that may be included within the invention as defined by the appended claims.

It is noted that, as used in this specification and the appended claims, the singular forms "a," "an" and "the" include plural referents unless expressly and unequivocally limited to one referent. Thus, for example, reference to "a sleeve" includes one, two, three or more sleeves.

We refer now to the drawings wherein depicted elements are not necessarily shown to scale and wherein like or similar elements are designated by the same reference numeral through the several views.

Referring to the drawings in general, it will be understood that the illustrations are for the purpose of describing particular embodiments of the disclosure and are not intended to be limiting thereto.

While most of the terms used herein will be recognizable to those of ordinary skill in the art, it should be understood that when not explicitly defined, terms should be interpreted as adopting a meaning presently accepted by those of ordinary skill in the art.

A capnography fitting is provided that is compatible with an inhalation mask (e.g., nasal mask) and can easily be inserted by both right handed and left-handed users. The capnography fitting provided fits inhalation masks of various sizes and shapes. In some embodiments, the capnography fitting provided allows the sampling tube to be easily inserted to and removed from the desired location in the inhalation mask.

In some embodiments, the present application provides, among other things, a capnography fitting that is designed to connect to an inhalation mask (e.g., nasal mask) so that viable carbon dioxide readings can be obtained from an air sample obtained from a patient's exhaled gas. The samples are then analyzed and displayed on the capnography device in order to track the patient's breathing patterns as a method to monitor the patient's breathing and respiratory status in real time.

Analyzing the capnogram generated from the capnography device may yield valuable information about the patient's clinical status. A normal capnogram exhibits one or more typical waveforms, each one represents a single respiratory cycle, and deviations from the normal waveform may indicate the clinical situation of the patient. For example, if the patient has reduced breathing during sedation, carbon dioxide will not be able to be exhaled by the patient. This lack of exhalation of carbon dioxide will make the capnogram show a low carbon dioxide reading, which will trigger an alarm (e.g., an audio and or visible alarm) that alerts medical staff to the problem.

In some embodiments, a capnography fitting is provided that is disposable for a single use. The fitting may be made of sterilizable materials and packaged with one or more disposable sample tubes such that a user can conveniently insert into the capnography fitting or install the capnography fitting into a circuit of a capnography system, which can allow easy change and disposal of the sample tube once used.

In some embodiments, the fitting can be used with a nasal mask available from Accutron, Inc. of Phoenix, Arizona in scented and unscented flavors and adult, large adult and pediatric sizes under the name ClearView^{™}. Suitable nasal masks for use with the capnography fitting are described in U.S. Patent No. 6,135,109 and U.S. Patent Publication No. 2017/0259018 assigned to Accutron, Inc. of Phoenix, Arizona. In some embodiments, nasal masks for use with the capnography fitting include Porter^{™} Brown two piece masks available from Porter Instrument, USA.

In some embodiments, the capnography fitting provides easy and reliable access to both sides of a nasal mask. The capnography fitting provided in the present application can be easily slidably installed into the nasal mask and does not require extensive manual modification by the user (e.g., placing holes in the nasal mask and/or tubing to obtain sampled air).

In some embodiments, the capnography fitting is reusable, and the capnography fitting can remain in a circuit of a capnography system for normal sterilization. In some embodiments, in the reusable configuration, the capnography fitting can be sterilized and reused while the sampling tube can be removed from the capnography fitting and disposed of when the procedure is completed.

### Capnography Fitting

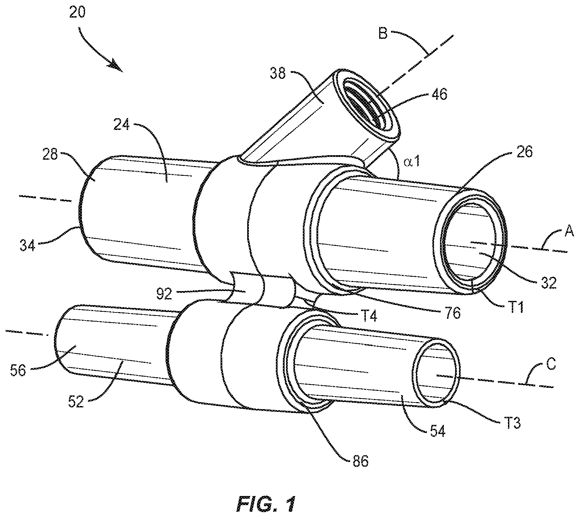

A disposable capnography fitting 20 is provided, as shown in FIGS. 1-13 that can be used with components of a capnography system 22. The fitting is suitable for universal use for both left handed and right-handed users and is configured for use with a variety of inhalation masks, such as for example nasal masks as described herein. The fitting provides an air-tight seal for gases entering and exiting the inhalation mask (e.g., nasal mask).

The fitting includes a first tube 24 having a proximal end 26, a distal end 28 and a longitudinal axis A disposed therebetween. More particularly, the first tube is configured to receive an inhalation gas, such as, for example, oxygen, carbon dioxide, nitrogen, an anesthetic gas, such as nitrous oxide, halogenated agents, such as, for example, halothane (Fluothane^{®}), enflurane (Ethrane^{®}), isoflurane (Forane^{®}), desflurane (Suprane^{®}), and sevoflurane (Ultane^{®}) or a combination thereof.

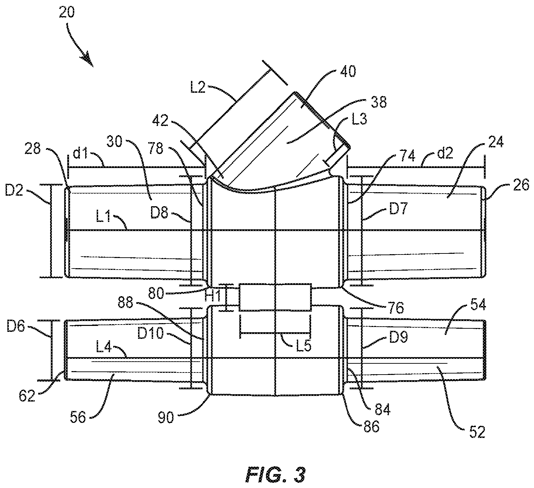

The first tube has a length L1, as shown in FIG. 3. Length L1 can be from about 20 to about 70 mm. The length L1 can be about 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70 mm.

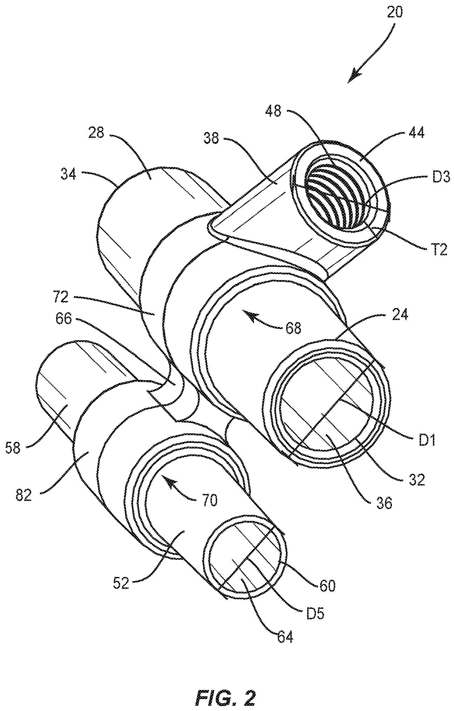

An exterior surface 30 of the first tube defines a first opening, such as a proximal end inlet 32 at the proximal end of the first tube. The proximal end inlet is configured to receive the inhalation gas, and to couple to (e.g., slidably engage) a sleeve, as described herein. The proximal end inlet includes a diameter D1, as shown in FIG. 2. Diameter D1 can have a diameter of from about 5 to about 15 mm. Diameter D1 can have a diameter of about 5, 6, 7, 8, 9, 10, 11, 12, 13, 14 or 15 mm.

The exterior surface of the first tube defines a second opening, such as a distal end outlet 34 at the distal end of the first tube. The distal end outlet is configured to couple with (e.g., slidably engage) an inlet of an inhalation mask, as described herein. The distal end outlet includes a diameter D2, as shown in FIG. 3. Diameter D2 can have a diameter of from about 5 to about 15 mm. Diameter D2 can have a diameter of about 5, 6, 7, 8, 9, 10, 11, 12, 13, 14 or 15 mm. In some embodiments, the diameter D1 of the proximal end inlet is equal to the diameter D2 of the distal end outlet. The diameter D1 of the proximal end inlet can, in some embodiments, be greater than or less than the diameter D2 of the distal end outlet. In the embodiment shown, the diameters D1 and D2 are the same and constant throughout the first tube.

An inner surface 36 extends between the proximal end and the distal end of the first tube which defines a hollow interior. The first tube can have the same or a varying thickness T1 throughout. For example, the thickness of the first tube can be the same or different at the proximal end and the distal end. The thickness T1 can be from about 0.1 mm to about 3 mm. The thickness can be about 0.1, 0.2, 0.3, 0.4, 0.5, 0.6, 0.7, 0.8, 0.9, 1, 2 or 3 mm.

The distal end outlet of the first tube is configured to slidably engage the inlet of the nasal mask and can be easily inserted into the inlet of a nasal mask and can provide a snug fit with the inlet. The capnography fitting can be removed from the mask once the procedure is over and, in some embodiments, discarded after use. In some embodiments, the first tube can be tapered at the proximal end and distal end. Alternatively, the proximal end or the distal end may be tapered.

The first tube includes an angled port 38. The angled port can be monolithic with the first tube and is configured to receive a sampling tube, as described herein. The angled port is in fluid communication with and disposed adjacent to the distal end outlet of the first tube. The angled port has a proximal end 40, a distal end 42 and an axis B which intersects longitudinal axis A of first tube 24. The angled port has a length L2 and L3, as shown in FIG. 3. Length L2 can be greater than length L3. Length L2 can be from about 10 to about 30 mm. Length L2 can be about 10, 11, 12, 13, 14, 15, 16, 17, 18, 19, 20, 21, 22, 23, 24, 25, 26, 27, 28, 29 or 30 mm. Length L3 can be from about 5 to about 20 mm. Length L3 can be about 5, 6, 7, 8, 9, 10, 11, 12, 13, 14, 15, 16, 17, 18, 19 or 20 mm.

The proximal end of the angled port includes opening 44 which has a diameter D3. Diameter D3 is less than or smaller than the diameter of D1 and D2. Diameter D3 can be from about 4 to about 10 mm. Diameter D3 can be about 4, 5, 6, 7, 8, 9 or 10 mm. It will be understood that the diameter of the angled port can also be the same diameter as the diameter of the first tube or a diameter larger than the diameter of the angled tube.

The opening of the angled port leads into an inner surface 46 that comprises threading 48. In some embodiments, the entire inner surface of the angled port is threaded or is at least partially threaded.

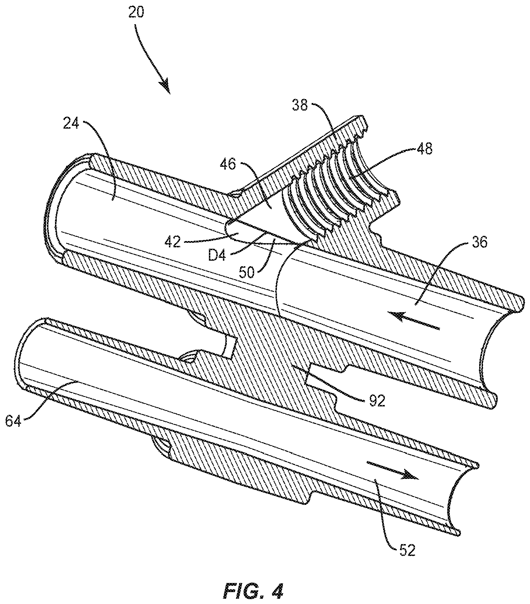

The distal end 42 of the angled port intersects with the first tube and allows gas flow in and/or out of the port. In some embodiments, the entire inner surface of the angled port is threaded or is at least partially threaded. The distal end of the port includes an opening 50 which has a diameter D4, as shown in FIG. 4. Like the port, opening 50 of the angled port is also angled. Opening 50 is an opening in the inner surface of the first tube that allows the port to intersect with the inner surface of the first tube. In this way, the angled port and the first tube are fluidly coupled and allow passage of gas into and out of the angled port and the first tube.

The angled port is ideal for inserting a sampling tube into the first tube and sampling gas, particularly gas exhaled by the patient and captured by the inhalation mask in order to obtain a capnography reading and monitor a patient's breathing pattern during the dental and/or medical procedure. Alternatively, the angled port can be ideal for sampling gas, particularly gas exhaled by the patient and captured by the inhalation mask from the port, without the need for a sampling tube, so that a capnography reading can be obtained directly from the angled port without the need to insert a sampling tube.

The bold arrows in FIG. 4 illustrate typical directions of gas flow in the first tube and the second tube. The angled port provides easy access to connect to a capnography device and provides an ideal area to sample carbon dioxide exhaled from the patient during respiration.

The angled port is disposed at an acute angle relative to the exterior surface of the proximal end inlet of the first tube, and has an angle α1 of from about 10 to about 60 degrees or from about 10 to about 89 degrees relative to the exterior surface of the first tube. In some embodiments, the angle α1 is from about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88 or 89 degrees relative to the exterior surface of the first tube. The angled port is configured to allow at least a portion of a sampling tube, as described herein, to be slidably received by the inner surface of the angled port, into the inner surface of the first tube to extend from the distal end outlet of the first tube and into an inlet of the inhalation mask (e.g., nasal mask). In some embodiments, the angled port is configured to connect to a capnography machine at the proximal end, as described herein.

By the port being angled, the user can slide a sampling tube into the port from the port opening. The sampling tube will be guided to the interior of the first tube and the sampling tube can extend out of the distal end of the first tube and be positioned in the inlet of the nasal mask and extend into the interior of the nasal mask and adjacent to the nostril of the patient, when the nasal mask is worn. In this way, the sampling tube, which is movable and not fixedly attached to the interior of the mask, will be adjacent to the nostril and the user can obtain a capnography reading from exhaled gas in real time.

In some embodiments, the angled port is spaced a greater distance d2 from the proximal end inlet than a distance d1 from the distal end outlet. In some embodiments, the angled port is spaced at an equal distance from the proximal end inlet to the distal end outlet.

The angled port has a thickness T2 that is greater than the thickness T1. In some embodiments, thickness T2 may be less than or equal to T1. The thickness T2 can be from about 0.2 mm to about 4 mm. The thickness can be about 0.2, 0.3, 0.4, 0.5, 0.6, 0.7, 0.8, 0.9, 1, 2, 3 or 4 mm. The fitting includes a second tube 52 adjacent to and extending substantially parallel to the first tube. The second tube includes a proximal end 54, a distal end 56 and a longitudinal axis C disposed therebetween. More particularly, the second tube is configured to receive exhalation gas from a patient, as described herein. The second tube has a length L4, as shown in FIG. 3. Length L4 is equal to the length of L1. Length L4 can be from about 20 to about 70 mm. The length L4 can be about 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70 mm.

An exterior surface 58 of the second tube defines a first opening, such as a proximal end outlet 60 at the proximal end of the second tube. The proximal end outlet is configured to slidably engage a sleeve, as described herein. The proximal end outlet includes a diameter D5, as shown in FIG. 2. Diameter D5 can be from about 3 to about 14 mm. Diameter D5 can be about 3, 4, 5, 6, 7, 8, 9, 10, 11, 12, 13 or 14 mm.

The exterior surface of the second tube defines a second opening, such as a distal end inlet 62 at the distal end of the first tube. The distal end inlet is configured for exhalation gas and to couple with an outlet of an inhalation mask, as described herein. The distal end inlet includes a diameter D6, as shown in FIG. 3. Diameter D6 can be from about 3 to about 14 mm. Diameter D6 can be about 3, 4, 5, 6, 7, 8, 9, 10, 11, 12, 13 or 14 mm. In some embodiments, the diameter D5 of the proximal end outlet is equal to the diameter D6 of the distal end inlet. The diameter D5 of the proximal end outlet can be greater than or less than the diameter D6 of the distal end inlet. The diameters D5 and D6 of the second tube are less than/smaller than diameters D1 and D2 of the first tube.

An inner surface 64 extends between the proximal end and the distal end of the second tube which defines a hollow interior. The second tube can have the same or a varying thickness T3 throughout. For example, the thickness of the second tube can be the same or different at the proximal end and the distal end.

The distal end inlet of the second tube is configured to slidably engage the outlet of the nasal mask and can be easily inserted into the outlet of the nasal mask and can provide a snug fit with the outlet. The capnography fitting can be removed from the mask once the procedure is over and, in some embodiments, discarded after use.

In some embodiments, the second tube can be tapered at the proximal end and the distal end. Alternatively, the proximal end or the distal end may be tapered.

According to the invention the first tube is fixed to the second tube by a support or web 66. The support comprises a first channel 68 configured to receive at least a portion of the first tube, and a second channel configured 70 configured to receive at least a portion of the second tube. The first tube and the second tube are held in a stacked configuration by the support. The support can be made from the same material or different material than the first tube and the second tube.

The support keeps the first and second tube in a fixed position and provides the stability to allow the user to insert the capnography fitting into the respective inlet and outlet of the inhalation mask. It also provides the stability to allow the user to insert the capnography fitting into the respective inlet and outlet of the sleeve.

The first channel of the support includes an exterior surface 72 that forms a first surface 74 having a first circular ledge 76 and a second surface 78 having a second circular ledge 80. The ledges are configured to prevent a sleeve and/or a mask attached to the fitting from translating onto the fitting beyond the ledges. The first surface of the first channel has a diameter D7 and the second surface of the first channel has a diameter D8. Diameters D7 and D8 can be equal, or different diameters. Diameters D7 and D8 can have a diameter of from about 6 to about 18 mm. Diameters D7 and D8 can have a diameter of about 6, 7, 8, 9, 10, 11, 12, 13, 14, 16, 17 or 18 mm. The diameter D1 and D2 of the first tube are less than diameters D7 and D8 of the first channel.

The second channel includes an exterior surface 82 that forms a first surface 84 having a first circular ledge 86 and a second surface 88 having a second circular ledge 90. The ledges are configured to prevent a sleeve and/or a mask attached to the fitting from translating onto the fitting beyond the ledges, as disclosed above. The first surface of the second channel has a diameter D9 and the second surface of the second channel has a diameter D10. Diameters D9 and D10 are equal, but, in some embodiments, can be different diameters.

Diameters D9 and D10 can be from about 4 to about 16 mm. Diameters D9 and D10 can be about 4, 5, 6, 7, 8, 9, 10, 11, 12, 13, 14, 15 or 16 mm. The diameters D5 and D6 of the second tube are less than diameters D9 and D10 of the second channel. Diameters D7 and D8 of the first channel are greater than diameters D9 and D10 of the second channel.

The support includes a link 92 that attaches the first channel with the second channel in the stacked configuration, as described above. The link can be formed from the exterior surfaces of the first and second channels. The link may also be variously shaped and configured. The link includes a length L5 and a height H1. The length L5 is greater than the height H1 of the link. The length L5 can be from about 5 mm to about 20 mm. The length L5 can be from about 5, 6, 7, 8, 9, 10, 11, 12, 13, 14, 15, 16, 17, 18, 19 or 20 mm. The height H1 can be from about 1 to about 10 mm. The height can be from about 1, 2, 3, 4, 5, 6, 7, 8, 9 or 10 mm. The link has a thickness T4 of from about 2 to about 8 mm. The link can have a thickness T4 of about 2, 3, 4, 5, 6, 7 or 8 mm.

The entire support, such as the first channel, the second channel and the link can be a single monolithic unit. Further, the entire fitting, such as the first tube, second tube, and entire support can be a single monolithic unit. Molding techniques can create a monolithic fitting. Alternatively, the fitting is not a single monolithic unit and the components are molded separately. Molding techniques include, but are not limited to blow molding, over-molding, injection molding, casting, machining, stamping, or any other suitable manufacturing process. Further, when the fitting is not monolithic, components of the fitting, as described herein, can alternatively be attached by any attachment means, such as, for example, via adhesive, glue, molding, over molding, curing with UV light, welding, ultrasonically welding, mechanical attachment, or the like or combinations thereof.

In some embodiments, the capnography fitting can be rigid or semi-rigid. In some embodiments, the capnography fitting can be flexible and have a modulus of elasticity of about 1 × 10² to about 6 × 10⁵ dynes/cm², or 2 × 10⁴ to about 5 × 10⁵ dynes/cm², or 5 × 10⁴ to about 5 × 10⁵ dynes/cm² or about 1 × 10² to about 6 × 10⁵ dynes/cm², or 2 × 10⁴ to about 5 × 10⁵ dynes/cm², or 5 × 10⁴ to about 5 × 10⁵ dynes/cm².

### Capnography System

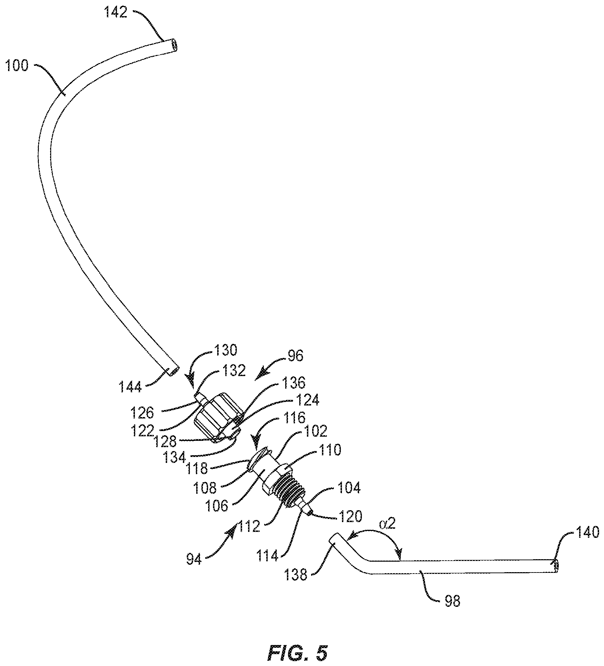

The angled port of the capnography fitting as described above, is configured to couple with a luer fitting, such as a female luer lock 94, an internally threaded cap 96, a sampling tube 98 and a return conduit 100, as shown in FIGS. 5-7. The luer fitting includes a proximal end 102 and a distal end 104. An exterior surface 106 defines a threaded portion 108 at the proximal end of the luer fitting. The threaded portion is configured to threadingly engage with an internally threaded portion of the internally threaded cap, as described below. The exterior surface 106 defines a flange 110, and at the distal end of the luer fitting, the exterior surface defines a threaded section 112 and a tapered section 114. The threaded section is configured for engagement with the threading on the inner surface of the angled port and the flange is configured for engagement with the proximal end of the port such that the luer fitting does not translate further into the angled port. A channel 116 having a first opening 118 and a second opening 120 is disposed longitudinally and through the center of the luer fitting.

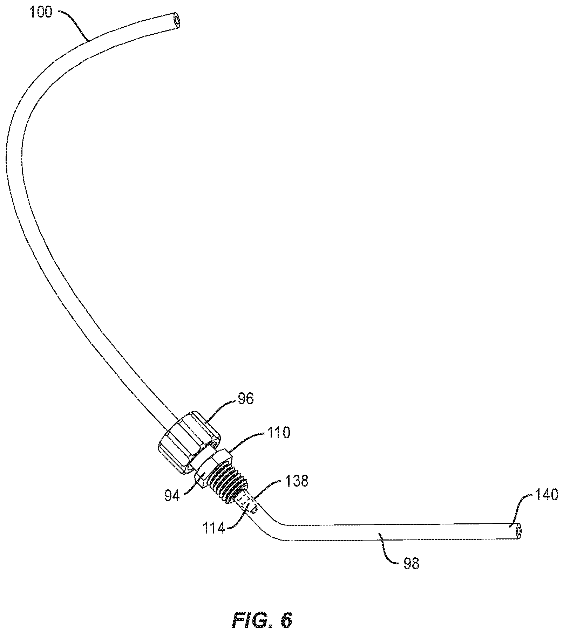

The internally threaded cap extends between a first end 122 and a second end 124. The first end includes a first tip 126 and the second end includes a second tip 128. A channel 130 having openings 132 and 134 is disposed longitudinally and through the center of the cap. An internally threaded surface 136 is configured to engage with the threaded portion of the luer fitting and the second tip is configured for disposal in the first opening of the luer fitting such that the internally threaded cap and the luer fitting couple and/or lock together, as shown in FIG. 6.

The sampling tube can be flexible and extends between a first end 138 and a second end 140. The first end of the sampling tube engages with the tapered section of the luer fitting. The sampling tube is configured to engage the angled port such that at least a portion of the sampling tube is slidably received by the inner surface of the angled port, into the inner surface of the first tube to extend from the distal end outlet of the first tube and into an inlet of the inhalation mask. The first end of the sampling tube has an angle α2 that can be from about 5 to about 60 degrees. In some embodiments, the angle α2 can be from about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 degrees.

The return conduit is flexible and extends between a first end 142 and a second end 144. The first end of the return conduit is configured to engage a portion of a capnography monitor and the second end of the return conduit is configured to engage the first tip of the internally threaded cap.

The sampling tube, as described herein, is configured to be slidably received by the inner surface of the angled port and can extend into the inner surface of the first tube and extend from the distal end outlet of the first tube and into an inlet of the inhalation mask (e.g., nasal mask). In some embodiments, the angled port is configured to connect to a capnography machine at the proximal end, as described herein.

By the port being angled, the user can slide a sampling tube into the port from the port opening. The sampling tube will be guided to the interior of the first tube and the sampling tube can extend out of the distal end of the first tube and be positioned in the inlet of the nasal mask and extend into the interior of the nasal mask and adjacent to the nostril of the patient, when the nasal mask is worn. In this way, the sampling tube, which is movable and not fixedly attached to the interior of the mask, will be adjacent to the nostril and the user can obtain a capnography reading from exhaled gas in real time.

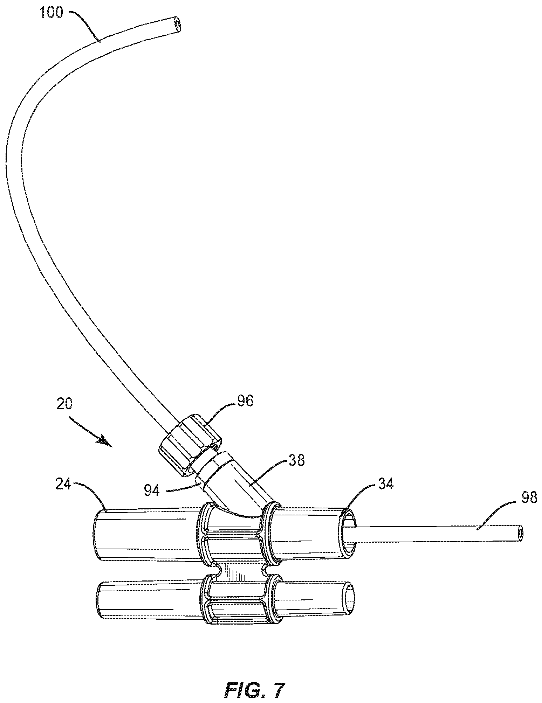

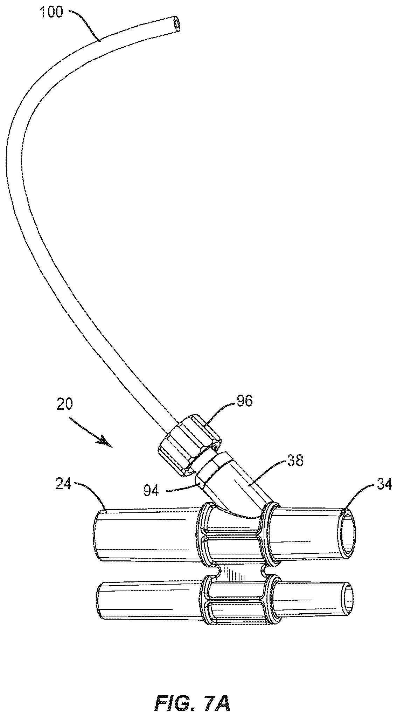

In some embodiments, as shown in FIG. 7A, a capnography reading can be obtained without using the sampling tube. For example, the return conduit is attached to a capnography monitor and the first tip of the internally threaded cap, as described above. The internally threaded cap engages the luer fitting which engages with the angled port. In this embodiment, the sampling tube is not attached to the luer fitting. In the embodiment shown in FIG. 7A, the angled port can be ideal for sampling gas, particularly gas exhaled by the patient and captured by the inhalation mask from the port, without the need for a sampling tube, so that a capnography reading can be obtained directly from the angled port without the need to insert a sampling tube.

In some embodiments, the length of the sampling tube and/or the return conduit can be cut into a desired length. The sampling tube can be cut into a desired length so as to access different areas of the mask in order to optimize a capnography reading such as a carbon dioxide reading. For example, an optimal reading can be obtained when the sampling tube has a longer length and the second end of the sampling tube is close to fresh gas.

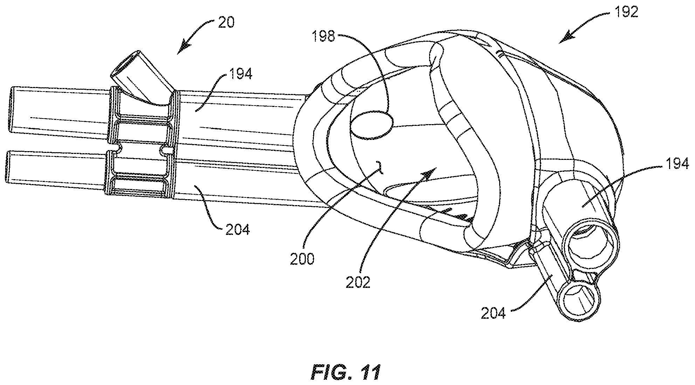

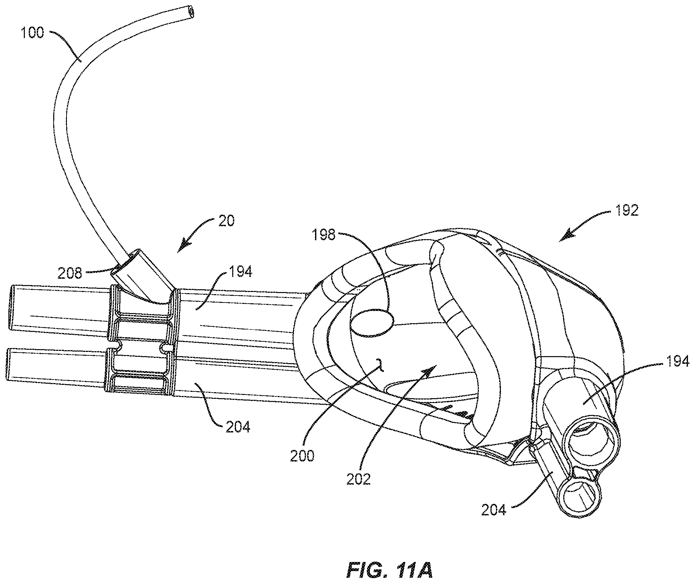

Referring to FIG. 11A, the sampling tube can alternatively engage the angled port via an interference fitting, press fitting or friction fitting 208. In this manner, the sampling tube is not attached to the luer fitting. Instead, the sampling tube has a slightly larger diameter or the same diameter as the angled port and is configured to be pressed or wedged into the opening at the proximal end of the angled port to provide a snug fit and the sampling tube is held in position by the angled port opening at the proximal end. In this embodiment, the sampling tube is still able to receive exhaled gas. In some embodiments, the sampling tube can also engage the angled port via a pressure fit, locking protrusion/recess, locking keyway and/or adhesive.

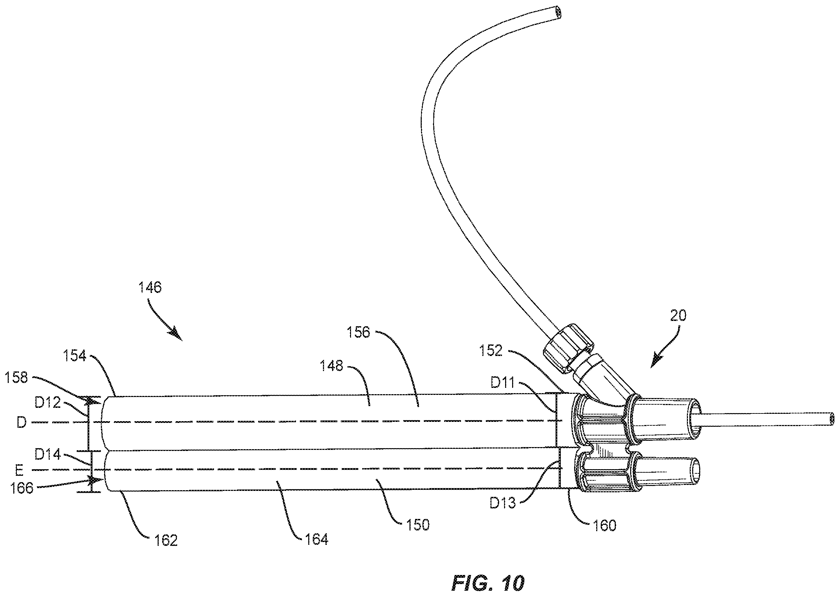

Referring to FIGS. 10-13, a sleeve 146, defines an upper section 148 and a lower section 150. The proximal end inlet of the first tube of the fitting and the proximal end outlet of the second tube of the fitting are configured to slidably engage the upper section and the lower section of the sleeve respectively. The sleeve can be bifurcated tubing that delivers respirable gas to the mask while also removing exhalation gas from the mask.

The upper section of the sleeve includes a proximal end 152, a distal end 154 and a longitudinal axis D disposed therebetween. The upper section of the sleeve includes an exterior surface 156 and an inner surface 158. The proximal end inlet of the first tube of the fitting is slidably engaged in the proximal end of the upper section of the sleeve and into a portion of the inner surface. The upper section of the sleeve at the proximal end has a diameter D11 and the distal end of the upper section of the sleeve has a diameter D12, as shown in FIG. 10. Diameters D11 and D12 are the same or equal. In some embodiments, diameters D11 and D12 are different. Diameters D11 and D12 can have a diameter of from 6 to about 20 mm. Diameters D11 and D12 can have a diameter of about 6, 7, 8, 9, 10, 11, 12, 13, 14, 15, 16, 17, 18, 19 or 20 mm. The diameter D11 is greater than D1 of the first tube of the fitting.

The lower section of the sleeve includes a proximal end 160, a distal end 162 and a longitudinal axis E disposed therebetween. The lower section of the sleeve includes an exterior surface 164 and an inner surface 166. The proximal end outlet of the second tube of the fitting is slidably engaged in the proximal end of the lower section of the sleeve and into a portion of the inner surface. The lower section of the sleeve at the proximal end has a diameter D13 and the distal end of the lower section of the sleeve has a diameter D14, as shown in FIG. 10. Diameters D13 and D14 are the same or equal. In some embodiments, diameters D13 and D14 are different. Diameters D13 and D14 can have a diameter of from 4 to about 18 mm. Diameters D13 and D14 can have a diameter of about 4, 5, 6, 7, 8, 9, 10, 11, 12, 13, 14, 15, 16, 17 or 18 mm. The diameter D13 is greater than D5 of the second tube of the fitting. The upper section of the sleeve diameters D11 and D12 have a diameter or diameters that are greater than diameters D13 and D14 of the lower section of the sleeve. The sleeve can be flexible and can have a modulus of elasticity that is less than the modulus of elasticity of the capnography fitting.

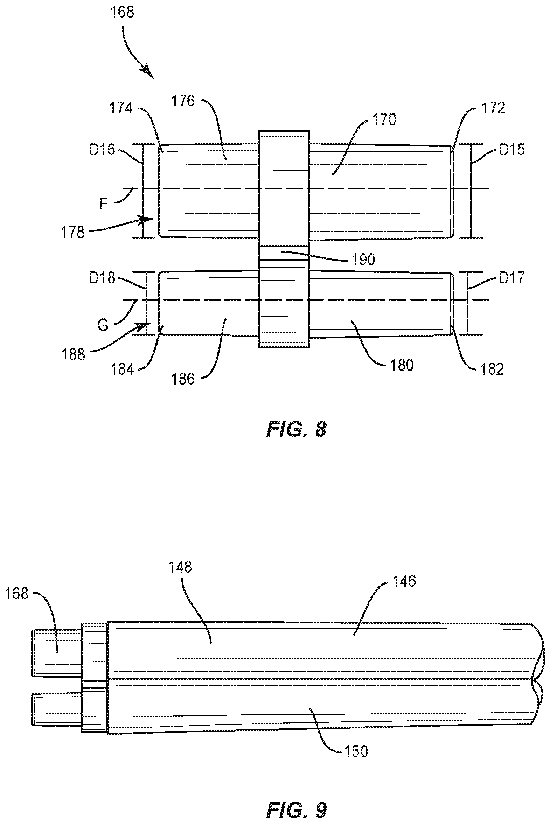

As shown in FIGS. 8 and 9, a second sleeve 146, having the same features as described above, is used in conjunction with a connector 168. The connector is similar to the connector assembly found and described in U.S. Patent No. 6,135,109, assigned to Accutron, Inc., which patent is incorporated herein by reference. The connector is configured to slidably engage the upper section and the lower section of the second sleeve and an opposing side of the mask that is not connected to the fitting, as described herein, to complete the capnography system circuit. Further, the connector is provided for admitting respirable gas into the mask and for conducting exhalation or exhaust gas from the mask. A suitable system operative for supplying a respirable gas to the mask and for providing a vacuum scavenger apparatus for collecting exhaust from the mask is fully disclosed in U.S. Pat. No. 5,311,862, of which is assigned to Accutron, Inc.

The connector includes a first tube 170 having a proximal end 172, a distal end 174 and a longitudinal axis F disposed therebetween. The first tube includes an exterior surface 176 and an inner surface 178. The proximal end of the first tube has a diameter D15 and the distal end of the first tube has a diameter D16, as shown in FIG. 8. The diameters D15 and D16 are the same as diameters D1 and D2 of the first tube of the fitting. Diameter D15 is less than diameter D11 of the upper section of the sleeve. The proximal end of the first tube of the connector is configured to slidably engage the proximal end of the upper section of the second sleeve and the distal end of the first tube of the connector is configured to engage a portion of a nasal mask, as described herein.

The connector includes a second tube 180 having a proximal end 182, a distal end 184 and a longitudinal axis G disposed therebetween. The second tube includes an exterior surface 186 and an inner surface 188. The proximal end of the second tube has a diameter D17 and the distal end of the second tube has a diameter D18, as shown in FIG. 8. The diameters D17 and D18 are the same as diameters D5 and D6 of the second tube of the fitting. Diameter D17 is less than diameter D13 of the lower section of the sleeve. The proximal end of the second tube of the connector is configured to slidably engage the proximal end of the lower section of the second sleeve and the distal end of the second tube of the connector is configured to engage a portion of a nasal mask, as described herein. The connector includes a support 190, similar or identical to support 66, as described above with regard to fitting 20, as shown in FIGS. 1-4.

As shown in FIGS. 11-14, a mask 192, such as a nasal mask is provided. The nasal mask is configured to administer respirable gas to a patient. Further, at least a portion of the sampling tube is configured to extend into the nasal mask, as described herein, such that a capnography reading can be taken close to the patient's nose for an accurate capnography reading. A nasal mask is available from Accutron, Inc. of Phoenix, Arizona in scented and unscented flavors and adult, large adult and pediatric sizes under the name ClearView^{™}. Suitable nasal masks for use with the capnography fitting are described in U.S. Patent No. 6,135,109 and U.S. Patent Publication No. 2017/0259018 assigned to Accutron, Inc. of Phoenix, Arizona. In some embodiments, nasal masks for use with the capnography fitting include Porter^{™} Brown two-piece masks available from Porter Instrument, USA.

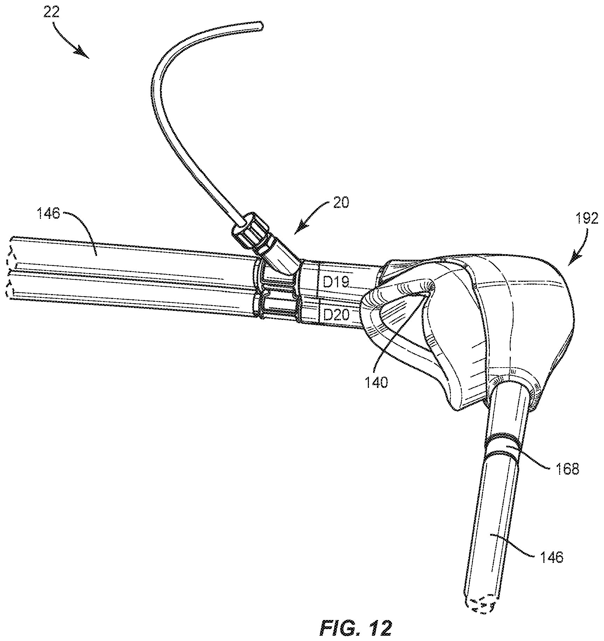

The nasal mask includes an inlet 194 that is configured for engagement with the distal end outlet of the first tube of the fitting, as shown in FIG. 11. The inlet has a diameter D19, as shown in FIG. 12 that is larger than the diameter D2 of the distal end of the first tube of the fitting. Diameter D19 can have a diameter of from about 6 to about 20 mm. Diameter D19 can have a diameter of about 6, 7, 8, 9, 10, 11, 12, 13, 14, 15, 16, 17, 18, 19 or 20 mm. An opening or inlet 198 is found on an interior surface 200 of the nasal mask and corresponds to the inlet. The sampling tube is slidably received by the inner surface of the angled port and into the inner surface of the first tube. The sampling tube then extends from the distal end outlet of the first tube and into the inlet of the nasal mask. From there, a portion of the sampling tube, such as the distal end of the sampling tube extends out of the inlet 198 and into a nasal chamber 202 of the nasal mask, as shown in FIGS. 10-12. A second inlet 194 is found in the same location but on the opposite side of the nasal mask, as shown in FIGS. 11-14.

The nasal mask includes an outlet 204 that is configured for engagement with the distal end inlet of the second tube of the fitting, as shown in FIG. 11. The outlet has a diameter D20 that is larger than the diameter D6 of the distal end of the second tube of the fitting. Diameter D20 can have a diameter of from about 4 to about 16 mm. Diameter D20 can have a diameter of about 4, 5, 6, 7, 8, 9, 10, 11, 12, 13 or 14 mm. A second outlet 204 is found in the same location but on the opposite side of the nasal mask, as shown in FIGS. 11-14.

The second inlet and outlet of the nasal mask is configured for attachment with the distal end of the first tube and the distal end of the second tube of the connector, as shown in FIGS. 12-14. The mask is disposable and is not reusable. The mask can also be various colors, sizes and scents.

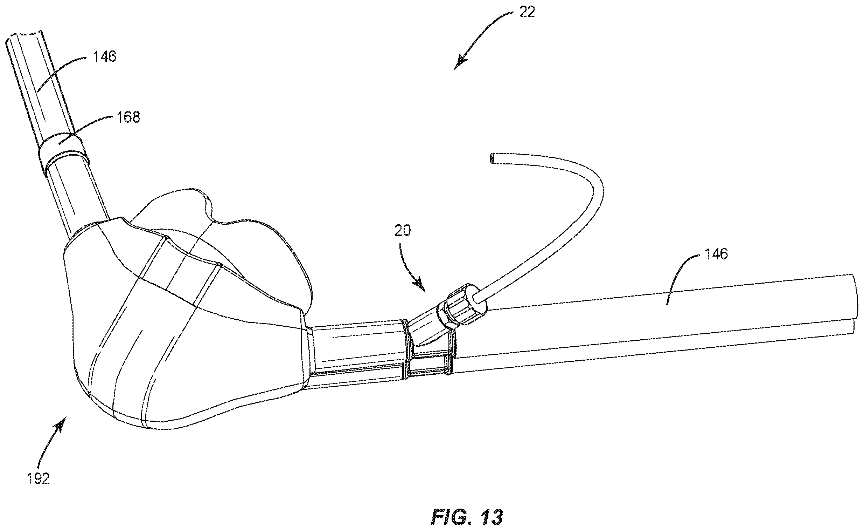

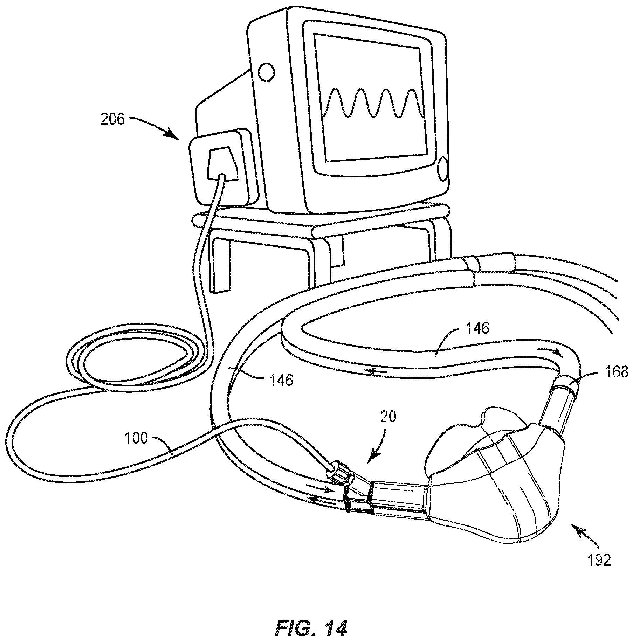

Referring to FIG. 13, the capnography fitting 20 is placed on the right side of the mask 192 and is ideal for right-handed clinicians to install the capnography fitting into the inlet and outlet of the mask. For left handed users, the capnography fitting 20 can be inserted in place of connector 168 on the left-hand side of the mask and the capnography fitting 20 can be switched with connector 168 on the right side of the mask as shown in FIG. 14. Thus, the capnography fitting 20 and connector 168 can easily be switched to the right side or left side of the mask based on the comfort of the clinician. Likewise, sampling of gas can be performed on the right or left side of the mask based on the comfort of the clinician.

Referring to FIG. 14, a capnography monitor 206 is provided. The capnography monitor is configured for engagement with the first end of the return conduit. In some embodiments, as described above, a portion of the capnography monitor can engage the first tip of the internally threaded cap directly without the use of the return conduit. The bold arrows in FIG. 14 illustrate typical directions of gas flow in the first tube and the second tube.

### Capnography Fitting Materials

The capnography fitting and/or components of the capnography system can be made from various materials. In some embodiments, the fitting and/or the components of the capnography system comprise a plastic material and can be a thermoplastic material. Suitable materials include, but are not limited to, polyurethane, polyurea, polyether(amide), PEBA, thermoplastic elastomeric olefin, copolyester, styrenic thermoplastic elastomer, carbon fiber, glass fiber, ceramics, methacrylates, poly (N-isopropylacrylamide), PEO-PPO-PEO (pluronics), rubber, plastic (e.g., polycarbonates), ABS, MABS, silicone, or the like or combinations thereof. In some embodiments, the components of the system can be made from a thermoplastic elastomer found under the trademark KRATON^{®}. In some embodiments, the capnography fitting and its components can be clear plastic.

In some embodiments, the capnography system can use various lubricants in order to assist in engagement of components of the system. In some embodiments, the lubricant comprises an oil lubricant. The oil lubricant can be polydimethyl siloxane, polytrifluoropropylmethyl siloxane, or a copolymer of dimethylsiloxane and trifluoropropylmethylsiloxane. In other embodiments, non-silicone based lubricants can be used that include, without limitation, a water soluble lubricant, an insoluble lubricant, a viscous gel lubricant, a solid lubricant or a combination thereof. Water soluble lubricants include, without limitation, polyethylene oxide, polyvinylpyrrolidone, polyvinyl alcohol, and derivatives thereof.

In various embodiments, non-silicone lubricants can be used, include hydrophilic polymer coatings, Teflon (PTFE) lubricants and coatings, thermoplastic coatings, cyanoacrylate coatings, Parylene coatings, plasma surface treatments, cornstarch powder coatings liquid soaps, Astroglide lubricants, mineral oil, glycerin, alcohol, saline, Krytox lubricants, molybdenum disulfide lubricants and graphite.

### Methods of Assembling and Use

A method of assembling a capnography system is provided, the method comprising: providing a capnography fitting, the capnography fitting comprising a first tube having a proximal end inlet and a distal end outlet, the first tube having an angled port in fluid communication with and disposed adjacent to the proximal end inlet and the distal end outlet, a second tube having a proximal end outlet and a distal end inlet, the second tube adjacent to and extending substantially parallel to the first tube and having a diameter smaller than a diameter of the first tube, and a support attached to the first tube and the second tube; inserting the proximal end inlet of the first tube and the proximal end outlet of the second tube into a sleeve; and coupling the distal end outlet of the first tube to an inlet of a nasal mask; and coupling the distal end inlet of the second tube to an outlet of the nasal mask to assemble the capnography system.

The sleeve defines an upper section and a lower section, the upper section having a diameter that is greater than a diameter of the lower section, as described above. During the method of assembling, the proximal end inlet of the first tube is inserted into the upper section of the sleeve and the proximal end outlet of the second tube is inserted into the lower section of the sleeve.

An inner surface of the angled port is configured to slidably receive at least a portion of a sampling tube, the sampling tube configured to extend out from the distal end outlet of the first tube into the inlet and a nasal chamber of the nasal mask, and the angled port is configured to couple with a luer fitting and an internally threaded cap.

A method of monitoring a patient's respiratory functions is provided, the method comprising: attaching a nasal mask to a nose of the patient, the nasal mask having an inlet to receive inhalation gas and an outlet to discharge exhalation gas, the nasal mask comprising a capnography fitting comprising a first tube having a proximal end inlet and a distal end outlet, the first tube having an angled port in fluid communication with and disposed adjacent to the proximal end inlet and the distal end outlet, a second tube having a proximal end outlet and a distal end inlet, the second tube adjacent to and extending substantially parallel to the first tube and having a diameter smaller than a diameter of the first tube, and a support attached to the first tube and the second tube, the distal end outlet of the first tube slidably engaging the inlet of the nasal mask and the distal end inlet of the second tube slidably engaging the outlet of the nasal mask; providing inhalation gas to the patient; and obtaining a sample of the exhalation gas from the angled port to monitor the patient's respiratory functions.

Referring to the method described above, an inner surface of the angled port is configured to slidably receive at least a portion of a sampling tube, the sampling tube configured to extend out from the distal end outlet of the first tube into the inlet of the nasal mask.

For the method of monitoring, at least a portion of the sampling tube extends into a nasal chamber of the nasal mask, and the patient's respiratory functions are patient breathing patterns and carbon dioxide levels produced from exhalations of the patient.

In some embodiments, a method of assembly is provided. The first step of the method includes removing the connector from the second sleeve. The second step includes inserting the proximal ends of the fitting into the proximal ends of the sleeve. The fitting should be assembled with the luer fitting, internally threaded cap, and sampling tube. The third step includes unwrapping and attaching a fresh disposable mask, such as a ClearView^{™} nasal hood mask onto the distal ends of the fitting, carefully guiding the sampling tube into the mask and attaching the connector/second sleeve to the opposing side of the mask. The inlet of the mask attaches to the distal end outlet of the first tube of the fitting and the outlet of the mask attaches to the distal end inlet of the second tube of the fitting. The fourth step includes confirming that a portion of the sampling tube is visible just inside the interior of the mask. The fifth step includes attaching the return conduit that is attached to the capnography monitor at its proximal end to the internally threaded cap attached to the luer fitting via the return conduit's distal end.

### Kits

A kit is provided for capnography monitoring, the kit comprising a capnography fitting comprising a first tube having a proximal end inlet and a distal end outlet, the first tube having an angled port in fluid communication with and disposed adjacent to the proximal end inlet and the distal end outlet, a second tube having a proximal end outlet and a distal end inlet, the second tube adjacent to and extending substantially parallel to the first tube and having a diameter smaller than a diameter of the first tube, and a support attached to the first tube and the second tube; and instructions for assembling the kit for capnography monitoring.

The kit further comprises a sleeve configured to slidably engage the proximal end inlet of the first tube and the proximal end outlet of the second tube; and a sampling tube being configured to extend out of the distal end outlet of the first tube.

A nasal mask can also be provided in the kit. The nasal mask has an inlet and an outlet, the inlet of the nasal mask configured to engage the distal end outlet of the first tube, and the outlet of the nasal mask configured to engage the distal end inlet of the second tube.

In various embodiments, the kit may include additional parts along with each component described in this disclosure, combined together to be used with the respective components. For example, the kit may include the capnography fitting in a first compartment. A second compartment may include the nasal mask. A third compartment may include the sleeves. A fourth compartment may include the connector. A fifth compartment may include the luer fitting, the internally threaded cap, the sampling tube and the return conduit. A sixth compartment may include a lubricant with or without gloves, drapes, wound dressings and other procedural supplies for maintaining sterility, as well as an instruction booklet. Each component of the kit (e.g., nasal mask, capnography fitting, connector, sampling tube, internally threaded cap, etc.) may be separately packaged in a plastic pouch that can be sterilized. A cover of the kit may include illustrations of the use of the device and a clear plastic cover may be placed over the compartments to maintain sterility.

The components of the kit for capnography monitoring may be lightweight, disposable and sterilizable. In various embodiments, one or more components of the kit can be sterilized by radiation in a terminal sterilization step in the final packaging. Terminal sterilization of a product provides greater assurance of sterility than from processes such as an aseptic process, which may require individual product components to be sterilized separately and the final package assembled in a sterile environment. Typically, in various embodiments, gamma radiation is used in the terminal sterilization step, which involves utilizing ionizing energy from gamma rays that penetrates into the components of the kit. Gamma rays are highly effective in killing microorganisms, they leave no residues nor have sufficient energy to impart radioactivity to the components of the kit. Gamma rays can be employed when the components of the kit are in the package and gamma sterilization does not require high pressures or vacuum conditions, thus, package seals and other components are not stressed. In addition, gamma radiation eliminates the need for permeable packaging materials.

In some embodiments, electron beam (e-beam) radiation may be used to sterilize one or more components of the kit. E-beam radiation comprises a form of ionizing energy, which is generally characterized by low penetration and high-dose rates. E-beam irradiation is similar to gamma processing in that it alters various chemical and molecular bonds on contact, including the reproductive cells of microorganisms. Beams produced for e-beam sterilization are concentrated, highly-charged streams of electrons generated by the acceleration and conversion of electricity.

Other methods may also be used to sterilize one or more components of the kit, including, but not limited to, gas sterilization, such as, for example, with ethylene oxide or steam sterilization.

Having now generally described the invention, the same may be more readily understood through the following reference to the following examples, which are provided by way of illustration and are not intended to limit the present invention unless specified.

### EXAMPLES

### Example 1: Port and Luer Lock Disposed in Tubing

The goal of this experiment was to provide a capnography fitting or connection in a low profile nasal hood (e.g., mask) system. The fitting or connection included a port having a female luer lock connection disposed on the outside of the tubing and near the mask. The purpose was to provide a suitable pathway to the mask where a reliable reading could be obtained. Alternatively, direct readings could be taken from the fitting alone and not from the luer lock.

A hole punch was used to create the hole in the tubing. The fitting was disposed in the hole and a small sampling tube was then passed into the fitting. A portion of the sampling tube was then inserted into the interior of the mask.

Results showed that a good capnography reading was obtained however, the port and luer lock of the fitting were hard to assemble and overall, the fitting was loose. Because of this, the fitting did not work well. Further, once the fitting was in the tube, it was impossible to add the sampling tube. Since the sampling tube could not be added after the fitting was disposed into the hole, researchers made a second attempt to attach the sampling tube to the fitting first. However, it was difficult to press the sampling into the tube properly. It was concluded that a different method or fitting was needed and it was suggested that a threaded luer lock might work correctly.

### Example 2: Current Capnography Fitting

After the first fitting was made in Example 1, the current capnography fitting as described above regarding capnography fitting 20 was made. The fitting was placed in the capnography system and a flow was set at 7.0 IPM. The vacuum was set to green (45 IPM). Results showed that the current capnography fitting worked and that clear signals were generated by a capnography monitor.

The present invention has been described above with reference to a preferred embodiment. However, those skilled in the art will recognize that changes and modifications may be made in the described embodiments without departing from the nature and scope of the present invention. Various changes and modifications to the embodiment herein chosen for purposes of illustration will readily occur to those skilled in the art. To the extent that such modifications and variations do not depart from the invention, they are intended to be included within the scope thereof.

## Claims

1. A capnography fitting comprising a first tube (24) having a proximal end inlet (32) configured to receive an inhalation gas and a distal end outlet (34) and a second tube (52) having a proximal end outlet (60) and a distal end inlet (62) configured to receive an exhalation gas, the second tube being adjacent to and extending substantially parallel to the first tube, wherein the first tube is fixed to the second tube by a support (66), the first tube having an angled port (38) in fluid communication with and disposed adjacent to the distal end outlet of the first tube and at an acute angle relative to a surface of the proximal end inlet of the first tube, the second tube having a diameter smaller than a diameter of the first tube, wherein the distal end outlet of the first tube is configured to couple with an inlet of an inhalation mask, and the distal end inlet of the second tube is configured to couple with an outlet of the inhalation mask and wherein an inner surface of the angled port is configured to slidably receive at least a portion of a sampling tube, which is configured to extend from the distal end outlet of the first tube into the inlet of the inhalation mask.

2. The fitting of claim 1, wherein the angled port has an angle of about 10 to about 60 degrees relative to the surface of the proximal end inlet of the first tube.

3. The fitting of claim 1, wherein the first tube and the angled port are monolithic.

4. The fitting of claim 1, wherein the angled port is spaced a greater distance from the proximal end inlet than a distance from the distal end outlet.

5. The fitting of claim 1, comprising a-sampling tube which is flexible and extends out from the distal end outlet of the first tube.

6. The fitting of claim 1, wherein the inner surface of the angled port has threading disposed thereon.

7. The fitting of claim 1, wherein the support comprises a first channel configured to receive at least a portion of the first tube and a second channel configured to receive at least a portion of the second tube and hold the first tube and the second tube in a stacked configuration.

8. The fitting of claim 1, wherein the proximal end inlet of the first tube and a proximal end outlet of the second tube are configured to slidably engage a sleeve.

9. The fitting of claim 8, comprising a sleeve which defines an upper section and a lower section, the upper section having a diameter that is greater than a diameter of the lower section.

10. The fitting according to any one of claims 1-9, wherein the first tube is configured to receive an inhalation gas comprising an anesthetic gas through the proximal end inlet of the first tube.

11. The fitting of claim 1, wherein the angled port is configured to couple with a luer fitting and an internally threaded cap.

## Patentansprüche

1. Capnographie-Armatur, umfassend ein erstes Rohr (24), das einen proximalen Endeinlass (32), der zum Empfangen eines Inhalationsgases konfiguriert ist, und einen distalen Endauslass aufweist: (34) und einen zweiten Schlauch (52), der einen proximalen Endauslass (60) und einen distalen Endeinlass (62) aufweist, der dazu konfiguriert ist, ein Exhalationsgas zu empfangen, wobei das zweite Rohr an das erste Rohr angrenzt und sich im Wesentlichen parallel zu diesem erstreckt, wobei das erste Rohr an dem zweiten Rohr durch eine Halterung (66) befestigt ist, wobei das erste Rohr einen abgewinkelten Anschluss (38) aufweist, der in Fluidverbindung mit dem distalen Endauslass des ersten Rohrs steht und angrenzend an diesen und in einem spitzen Winkel relativ zu einer Oberfläche des proximalen Endeinlasses des ersten Rohrs angeordnet ist, wobei das zweite Rohr einen Durchmesser aufweist, der kleiner ist als der Durchmesser des ersten Rohrs, wobei der distale Endauslass des ersten Rohrs dazu konfiguriert ist, mit einem Einlass einer Inhalationsmaske zu koppeln, und der distale Endeinlass des zweiten Rohrs dazu konfiguriert ist, mit einem Auslass der Inhalationsmaske zu koppeln, und wobei eine Innenfläche der abgewinkelten Öffnung dazu konfiguriert ist, mindestens einen Abschnitt eines Probennahmerohrs gleitend zu empfangen, das dazu konfiguriert ist, sich von dem distalen Endauslass des ersten Rohrs in den Einlass der Inhalationsmaske zu erstrecken.

2. Armatur nach Anspruch 1, wobei die abgewinkelte Öffnung einen Winkel von etwa 10 bis etwa 60 Grad relativ zu der Oberfläche des proximalen Endeinlasses des ersten Rohrs aufweist.

3. Armatur nach Anspruch 1, wobei das erste Rohr und die abgewinkelte Öffnung monolithisch sind.

4. Armatur nach Anspruch 1, wobei die abgewinkelte Öffnung in einem größeren Abstand zu dem proximalen Endeinlass angeordnet ist als zu dem distalen Endauslass.

5. Armatur nach Anspruch 1, umfassend ein Probennahmerohr, das flexibel ist und sich von dem distalen Endauslass des ersten Rohrs aus erstreckt.

6. Armatur nach Anspruch 1, wobei die Innenfläche der abgewinkelten Öffnung ein Gewinde darauf aufweist.

7. Armatur nach Anspruch 1, wobei die Halterung einen ersten Kanal umfasst, der dazu konfiguriert ist, mindestens einen Abschnitt des ersten Rohrs zu empfangen, und einen zweiten Kanal, der dazu konfiguriert ist, mindestens einen Abschnitt des zweiten Rohrs zu empfangen und das erste Rohr und das zweite Rohr in einer gestapelten Konfiguration zu halten.

8. Armatur nach Anspruch 1, wobei der proximale Endeinlass des ersten Rohrs und ein proximaler Endauslass des zweiten Rohrs dazu konfiguriert sind, gleitend in eine Hülse einzugreifen.

9. Armatur nach Anspruch 8, umfassend eine Hülse, die einen oberen Abschnitt und einen unteren Abschnitt definiert, wobei der obere Abschnitt einen Durchmesser aufweist, der größer ist als der Durchmesser des unteren Abschnitts.

10. Armatur nach einem der Ansprüche 1-9, wobei das erste Rohr dazu konfiguriert ist, ein Inhalationsgas, das ein Anästhesiegas umfasst, durch den proximalen Endeinlass des ersten Rohrs zu empfangen.

11. Armatur nach Anspruch 1, wobei die abgewinkelte Öffnung dazu konfiguriert ist, mit einer Luer-Armatur und einer Kappe mit Innengewinde zu koppeln.

## Revendications

1. Raccord de capnographie comprenant un premier tube (24) présentant une entrée d'extrémité proximale (32) configurée pour recevoir un gaz d'inhalation et une sortie d'extrémité distale : (34) et un second tube (52) présentant une sortie d'extrémité proximale (60) et une entrée d'extrémité distale (62) configurée pour recevoir un gaz d'expiration, le second tube étant adjacent et se prolongeant sensiblement parallèlement au premier tube, dans lequel le premier tube est fixé au second tube par un support (66), le premier tube présentant un orifice incliné (38) en communication fluidique avec et disposé adjacent à la sortie d'extrémité distale du premier tube et à un angle aigu par rapport à une surface de l'entrée d'extrémité proximale du premier tube, le second tube présentant un diamètre inférieur à un diamètre du premier tube, dans lequel la sortie d'extrémité distale du premier tube est configurée pour se coupler avec une entrée d'un masque d'inhalation, et l'entrée d'extrémité distale du second tube est configurée pour se coupler avec une sortie du masque d'inhalation et dans lequel une surface interne de l'orifice incliné est configurée pour recevoir de manière coulissante au moins une partie d'un tube d'échantillonnage, qui est configuré pour se prolonger depuis la sortie d'extrémité distale du premier tube jusqu'à l'entrée du masque d'inhalation.

2. Raccord selon la revendication 1, dans lequel l'orifice incliné présente un angle d'environ 10 à environ 60 degrés par rapport à la surface de l'entrée d'extrémité proximale du premier tube.

3. Raccord selon la revendication 1, dans lequel le premier tube et l'orifice incliné sont formés d'un seul tenant.

4. Raccord selon la revendication 1, dans lequel l'orifice incliné est espacé d'une plus grande distance de l'entrée d'extrémité proximale que d'une distance de la sortie d'extrémité distale.

5. Raccord selon la revendication 1, comprenant un tube de prélèvement qui est souple et se prolonge vers l'extérieur depuis la sortie de l'extrémité distale du premier tube.

6. Raccord selon la revendication 1, dans lequel la surface interne de l'orifice incliné présente un filetage disposé sur celle-ci.

7. Raccord selon la revendication 1, dans lequel le support comprend un premier canal configuré pour recevoir au moins une partie du premier tube et un second canal configuré pour recevoir au moins une partie du second tube et maintenir le premier tube et le second tube dans une configuration empilée.

8. Raccord selon la revendication 1, dans lequel l'entrée d'extrémité proximale du premier tube et une sortie d'extrémité proximale du second tube sont configurées pour entrer en prise de manière coulissante avec un manchon.

9. Raccord selon la revendication 8, comprenant un manchon qui définit une section supérieure et une section inférieure, la section supérieure présentant un diamètre supérieur à un diamètre de la section inférieure.

10. Raccord selon l'une quelconque des revendications 1 à 9, dans lequel le premier tube est configuré pour recevoir un gaz d'inhalation comprenant un gaz anesthésique à travers l'entrée d'extrémité proximale du premier tube.

11. Raccord selon la revendication 1, dans lequel l'orifice incliné est configuré pour se coupler avec un raccord Luer et un capuchon fileté intérieurement.
